# EUROPEAN PATENT SPECIFICATION

(11) **EP 2 528 596 B1**
(45) Date of publication and mention of the grant of the patent: **29.06.2016**
(21) Application number: 11708943.3
(22) Date of filing: 28.01.2011
(51) Int. Cl.: A61K 31/13, A61K 31/381, A61K 31/575, A61K 9/00, A61P 11/00, A61P 11/06

(54) **DRY POWDER FORMULATIONS ADMINISTERED BY THE INHALATION ROUTE COMPRISING A COMBINATION OF TIOTROPIUM BROMIDE, SALMETEROL XYNAFOATE AND FLUTICASONE PROPIONATE.**
ZUSAMMENSETZUNG IN TROCKENPULVERFORM ZUM INHALIEREN ENTHALTEND EINE KOMBINATION AUS TIOTROPIUM BROMIDE, SALMETEROL XYNAFOATE UND FLUTICASONE PROPIONATE.
COMPOSITION SOUS FORME DE POUDRE SÈCHE POUR ADMINISTRATION PAR INHALATION COMPRENANT UNE COMBINAISON DE BROMURE DE TIOTROPIUM, DE XYNAFOATE DE SALMETEROL ET DE PROPIONATE DE FLUTICASONE.

(30) Priority: 29.01.2010 TR 201000681
(43) Date of publication of application: 05.12.2012
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2011/000023
(87) International publication number: WO 2011/105975

(56) References cited:
- EP-A1- 0 418 716
- WO-A1-2004/019985
- WO-A1-2005/053648
- WO-A1-2010/007446
- WO-A2-2006/086130
- US-A1- 2003 018 019
- US-A1- 2004 009 963
- SINGH D ET AL: "Superiority of triple'' therapy with salmeterol/fluticasone propionate and tiotropium bromide versus individual components in moderate to severe COPD", THORAX, BMJ PUBLISHING GROUP, GB, vol. 63, no. 7, 1 July 2008 (2008-07-01), pages 592-598, XP009147851, ISSN: 0040-6376, DOI: DOI:10.1136/THX.2007.087213 [retrieved on 2008-02-01]
- CAZZOLA ET AL: "A pilot study to assess the effects of combining fluticasone propionate/salmeterol and tiotropium on the airflow obstruction of patients with severe-to-very severe COPD", PULMORNARY PHARMACOLOGY & THERAPEUTICS, ACADEMIC PRESS, GB, vol. 20, no. 5, 20 July 2007 (2007-07-20), pages 556-561, XP022162533, ISSN: 1094-5539, DOI: DOI:10.1016/J.PUPT.2006.06.001
- JAMES J ET AL: "The surface characterisation and comparison of two potential sub-micron, sugar bulking excipients for use in low-dose, suspension formulations in metered dose inhalers", INTERNATIONAL JOURNAL OF PHARMACEUTICS, ELSEVIER BV, NL, vol. 361, no. 1-2, 1 September 2008 (2008-09-01), pages 209-221, XP023316079, ISSN: 0378-5173, DOI: DOI:10.1016/J.IJPHARM.2008.05.032 [retrieved on 2008-06-04]

## Description

### DESCRIPTION

The present invention relates to a pharmaceutical composition comprising tiotropium, salmeterol, fluticasone, and/or pharmaceutically acceptable derivatives thereof and the use of this composition in the treatment of respiratory diseases particularly in asthma, allergic rhinitis and chronic obstructive pulmonary diseases (COPD).

Airways, in other words bronchia, are the channels which function to distribute the inhaled air into the lung tissues. In the case of respiratory diseases such as asthma or chronic obstructive pulmonary disease (COPD), stimulants such as allergen, infection, good and bad smell, smoke, genetic factors and exercise cause contractions in the airway muscles (bronchoconstruction) and/or excessive secretion in glands and results in contractions in the airway. Hence, respiration gets more difficult as the inhaled air cannot be exhaled.

Corticosteroids which are used in the treatment of asthma and COPD are synthetic and strong anti-inflammatory drugs that are similar to natural corticosteroid hormones produced by adrenal glands. They prevent both the transcription of the inflammatory gene and the activation of the anti-inflammatory gene. In addition to this, they increase the transcription of β₂ receptors. Furthermore, corticosteroids prevent the tolerance that develops after a long-term application of β₂ agonists. Beclomathasone, budesonide, ciclesonide, flunisolide, fluticasone are among the steroids that are used for the treatment of respiratory diseases.

Corticosteroids are not preferred in acute asthma crises as they do not have rapid onset of action. Since inhaled corticosteroids may prevent growth in children, they are advised to be used in the lowest possible amount. Long-term use of corticosteroids may cause cataract and glaucoma. In addition, they may cause some serious side-effects such as osteoporosis, high cholesterol, edema, encepholalgia, weight gain, insomnia and some skin problems.

β₂ adrenergic agonists which are used in the treatment of respiratory diseases such as asthma and COPD affect the muscles around the air vessels by activating β₂ adrenergic receptors. They reduce or eliminate bronchospasm. Bronchodilator β₂-agonists are categorized into two groups as long-acting and short-acting. Short-acting beta-agonists such as salbutamol, levosalbutamol, prosaterol, fenoterol, terbutaline, pirbuterol, metoproterenol, bitolterol mesilate have a rather rapid onset of action such as 3-5 minutes and their duration of action is of 4-6 hours. As they have a rapid onset of action, they are given as relaxant but they should be taken very often since they have a short duration of action. Long-acting β₂ agonists have a slower onset of action compared to the other group but their duration of action is of 12 hours. Salmeterol, formoterol, bambuterol and clenbuterol can be given as examples to long-acting β₂ agonists. Long-acting β₂-agonists are often used in the treatment of patients who present asthma symptoms at nights and in the treatment of asthma stimulated by exercise.

β₂ agonists are known as the most effective agents in order to eliminate acute asthma symptoms. One of the most important factors for their being used as symptomatolytic is their onset of action. Some long-acting β₂ agonists can have the onset of action of short acting β₂ agonists when used at specific doses.

β₂ agonists affect the muscles above the air vessels. However, they might affect the muscles around the heart and the bones. In the case that they affect the heart muscles, acceleration of heartbeat and palpitation might be observed. In 2005, US Food and Drug Administration (FDA) revealed that many long-acting β₂-agonists drugs increase wheezing symptom in patients. Following this, in a study carried out by Cornell and Stanford Universities, it was found out that regular intake of β-agonists in the treatment of COPD increases health problems resulting from respiratory tract.

Anticholinergics are the other active agents which are utilized in the treatment of respiratory diseases. Anticholinergics influence large airways including the muscles above bronchia, while β₂ agonists influence small airways, in other words bronchioles. Anticholinergics such as β₂ agonists are categorized into two groups as long-acting and short-acting. Short-acting anticholinergics containing ipratropium bromide and oxitropium bromide have an onset of action of 15 minutes and their duration of action is 6-8 hours. The long-acting anticholinergic agent is tiotropium. Tiotropium has an onset of action of 20 minutes and its duration of action is 24 hours. Thus, it is enough to take it once a day. Side effects of anticholinergics are weaker than the side effects of β₂ agonists.

The use of combination drugs in the treatment of respiratory diseases such as asthma and COPD is very effective particularly in decreasing asthma attacks. It is possible that the severity or occurrence possibility of the abovementioned side effects decreases as the active substances that are used in combinations are more effective at lower doses compared to the active substances used alone.

In this context the disclosure of US2003018019 is relevant.

However, decreasing the side effects that arise from the active agents is not sufficient to provide effective treatment for respiratory diseases. The medicaments used in the formulations should be selected in a way to give the best combination and furthermore, they should be in the most stable form. Moreover, the compositions comprising them should be formulated in such a way that the composition is stable and also it reaches to the target area in the most efficient way.

The inventor has surprisingly found that unexpected therapeutic benefits are obtained through the use of the combination comprising tiotropium bromide with water content less than or equal to 2.5%, salmeterol xinafoate and fluticasone or pharmaceutically acceptable derivatives thereof separately or together for simultaneous or sequential administration in the prevention or treatment of respiratory diseases.

It was seen that a combination comprising tiotropium bromide with water content less than or equal to 2.5%, salmeterol xinafoate and fluticasone propionate provides the most stable and therapeutically beneficial combination for simultaneous or sequential administration in the prevention or treatment of respiratory diseases. Preferably, a composition comprising tiotropium bromide with water content less than or equal to 2.5%, salmeterol xinafoate and fluticasone propionate is administered simultaneously. In another aspect, said composition comprises tiotropium bromide with water content less than or equal to 2.5%, formoterol fumarate, salmeterol xinafoate and lactose.

In another aspect, tiotropium bromide with water content less than or equal to 2.5%, salmeterol xinafoate, formoterol fumarate are preferably combined in a single dosage form.

Furthermore, the amount of the active agents used in the composition is carefully adjusted in order to prevent the side effects that might arise from these agents and it was seen that minimum side effects are observed when tiotropium bromide with water content less than or equal to 2.5%: salmeterol xinafoate: fluticasone propionate ratio in the composition is in the range of 1 : 1 : 1 to 1 : 50 : 50 by weight. Furthermore, it was seen that in the formulation, that is constituted with active agents in the amounts which have the ratio in the range of 1 : 1 : 1 to 1 : 50 : 50 , the adhesive force between the particles is less and hence, the amount of inhaled particles and efficacy of the formulation increases.

Accordingly; the present invention provides a combined drug preparation comprising tiotropium bromide with water content less than or equal to 2.5%, salmeterol xinafoate and fluticasone propionate in amounts in the range of 1 : 1 : 1 to 1 : 50 : 50 for simultaneous or sequential administration in the prevention or treatment of respiratory diseases such as asthma and chronic obstructive pulmonary diseases (COPD).

According to another aspect, the present invention provides an effective inhalation of the drug comprising tiotropium bromide with water content less than or equal to 2.5%, salmeterol xinafoate and fluticasone propionate.

The drug pertaining to the present invention is preferred to be administered by the inhalation route as it a) has a more rapid onset of action compared to the administration via oral or parenteral routes b) enables the use at lower doses c) minimizes the side effects.

According to another aspect, the present invention provides simultaneous or sequential inhalation of the drug comprising tiotropium bromide with water content less than or equal to 2.5%, salmeterol xinafoate and fluticasone propionate by the inhalation route.

According to another aspect, the present invention provides the transmission of the drug comprising tiotropium bromide with water content less than or equal to 2.5%, salmeterol xinafoate and fluticasone propionate via single or multi dose inhalers.

The drug comprising tiotropium bromide with water content less than or equal to 2.5%, salmeterol xinafoate and fluticasone propionate can be in dry powder form; they can be formulated with propellant gases to give aerosol formulations or they can be formulated with solvents to give nebulizer formulations. The inventors have found that the best way to transfer the medicament comprising tiotropium bromide with water content less than or equal to 2.5%, salmeterol xinafoate and fluticasone propionate is using this medicament combination in dry powder form.

In this way, the components maintain their stability and furthermore the medicament is in a stable form and is easily used by the patients.

In order to ensure effective absorption of the active agents into the lung tissue, the particle size of the agents should be adjusted. Although large particle size provides ease in manufacturing the dry powder, it may accumulate in throat and lead to insufficient intake of the medicament. Very fine particles, on the other hand, may reach the lungs. However, they might not have a good flow property which in turn causes problems in providing dose accuracy. To prevent these problems, the active agents should have an optimum average particle size. The inventors have found that tiotropium bromide with water content less than or equal to 2.5%, salmeterol xinafoate, fluticasone propionate having a mean particle size in the range of 1.5 to 4.5 µm reaches lungs effectively and also no problems related to flow properties of the dry powder are observed.

In one aspect, the present invention provides a medicament composition comprising tiotropium bromide with water content less than or equal to 2.5%, salmeterol xinafoate and fluticasone propionate in dry powder form wherein the mean particle size of tiotropium bromide with water content less than or equal to 2.5%, salmeterol xinafoate and fluticasone propionate is in the range of 1.5 to 4.5 µm and wherein said active agents are present in the composition with the ratio of 1 : 1 : 1 to 1:50:50 respectively and said composition can be simultaneously or sequentially administered in the prevention or treatment of respiratory diseases.

The term "mean particle size" refers to particles wherein the particle size of 50% of the total number of particles is less than the average particle size.

According to the present invention, the drug comprising tiotropium bromide with water content less than or equal to 2.5%, salmeterol xinafoate and fluticasone propionate may also contain effective amounts of excipients and/or additional agents apart from active agents.

According to the present invention, the dry powder formulation comprising tiotropium bromide with water content less than or equal to 2.5%, salmeterol xinafoate and fluticasone propionate is transmitted to the patient in dry powder form. Said dry powder formulations also contain some physiologically acceptable excipients along with the active agent. These excipients can be monosaccharides (glucose, etc.), disaccharides (lactose, saccharose, maltose, etc.), oligosaccharides and polysaccharide (dextran, etc.), polyalcohols (sorbitol, mannitol, xylitol, etc.), salts (sodium chloride, calcium carbonate, etc.) or a mixture thereof.

The inventors have found that in compositions according to present invention, in other words in compositions comprising tiotropium bromide with water content less than or equal to 2.5%, salmeterol xinafoateand fluticasone propionate in dry powder form wherein the mean particle size of tiotropium bromide with water content less than or equal to 2.5%, salmeterol xinafoate and fluticasone propionate is in the range of 1.5 to 4.5 µm and wherein said active agents are present in the composition with the ratio of 1 : 1 : 1 to 1 : 50 : 50 respectively, using lactose as the one and only carrier provides optimum homogeneity and flow properties to the dry powder and therefore, dose accuracy is maintained.

It was also seen that the mean particle size of the carrier plays an important role in the delivery of the medicament to the target area, i.e. lungs, effectively in the compositions pertaining to the present invention. It was found that the adhesive forces present between the lactose particles and the active agents having the mean particle size in the range of 1.5 to 4.5 µm are minimized and thus an effective inhalation of the active agents takes place when lactose having a mean particle size less than or equal to 100 µm is used in compositions comprising tiotropium bromide with water content less than or equal to 2.5%, salmeterol xinafoate and fluticasone propionate in dry powder form wherein the mean particle size of tiotropium bromide with water content less than or equal to 2.5%, salmeterol xinafoateand fluticasone propionate is in the range of 1.5 to 4.5 µm and wherein said active agents are present in the composition with the ratio of 1 : 1 : 1 to 1 : 50 : 50 respectively.

In another aspect, the present invention provides a composition comprising tiotropium bromide with water content less than or equal to 2.5%, salmeterol xinafoate and fluticasone propionate in dry powder form wherein;
- the mean particle size of tiotropium bromide with water content less than or equal to 2.5%, salmeterol xinafoate and fluticasone propionate is in the range of 1.5 to 4.5 µm and
- said active agents are present in the composition with the ratio of 1 : 1 : 1 to 1 : 50 : 50 respectively and
- lactose which has a mean particle size less than 100 µm is used as carrier.

Lactose which has a mean particle size less than 100 µm is preferably used as a mixture of particles having two different mean particle sizes. Accordingly, lactose which has mean particle size less than 100 µm can be present as a mixture of particles having a mean particle size less than 10 µm (fine) and particles having a mean particle size in the range of 10 µm to 100 µm (coarse). The inventors have observed that the adhesive force between the active agents and lactose is even less when lactose which has two different mean particle sizes is used.

The ratio of lactose which has a mean particle size less than 10 µm (fine) to lactose which has a mean particle size in the range of 10 µm to 100 µm (coarse) is in the range of 1:1 to 1:25, preferably in the range of 1:1 to 1:10, most preferably in the range of 1 : 1,5 to 1: 5.

According to the present invention, the amount of pharmaceutically acceptable carrier is preferably in the range of 0-50 mg.

According to another aspect, the present invention provides a method to transmit the drug combination comprising tiotropium, salmeterol and fluticasone and/or pharmaceutically acceptable derivatives thereof via a dry powder inhaler in which the drug is stored in peelable blister packs, capsules or a reservoir for use in the treatment of patients suffering from respiratory diseases.

In the inhalation devices which are designed to transmit dry powder drugs, an effective amount of the dry powder drug is prepared for inhalation when the device is triggered.

In order to prepare the dry powder formulation stored in capsules, the supplementary components in the device provides the capsule to open or be pierced when the device is triggered and the dry powder formulation is prepared for inhalation. After the inhalation is completed, the empty capsule is ejected from the device and a new capsule is placed immediately before the following inhalation takes place.

According to the present invention, the capsule can be made of a substance chosen from a group comprising gelatine, chitosan, starch and/or starch derivatives, cellulose and/or cellulose derivatives or synthetic polymers as well as consisting intertwined upper and lower compartments.

In the case that the capsule used in the present invention is made of cellulose or its derivatives, the capsule material can be selected from, but not limited to, a group comprising hydroxypropyl cellulose, hydroxypropylmethyl cellulose, methyl cellulose, hydroxymethyl cellulose, hydroxyethyl cellulose.

In the case that the capsule used in the present invention is synthetic polymer, the capsule material can be selected from, but not limited to, a group comprising polyethylene, polyetheleneteraphtalate, polycarbonate or polypropylene.

In the case that the capsule material used in the present invention is gelatine, additional agents such as polyethylene glycol, sorbitol, glycerol, propylene glycol, polyethylene oxide - polypropylene oxide block copolymers and/or other polyalcohols or polyethers at different molecular weights can be added into it.

The dry powder drug pertaining to the present invention can also be stored in blister packs apart from reservoirs and capsules. Blister packs are comprised of orderly placed blisters each of which contains minimally one dose of the dry powder drug. Blister packs can be pierced or peeled to be opened according to the device design. However, peelable blister packs are preferred according to the present invention. When the device is triggered, the blister pack or one of the blisters in the pack is pierced or peeled and the drug in dry powder form is prepared for inhalation.

The cavity volume of blisters pertaining to the present invention, which are placed side by side in an order and which provide to transmit and store the dry powder drug in blister pack is in the range of 17-30 mm³, preferably in the range of 18-23 mm³, most preferably in the range of 19-21 mm^{3.}

The cavity volume of the blisters pertaining to the present invention, which provide to transmit and store the dry powder drug comprising tiotropium bromide having water content less than 2.5%, salmeterol xinafoate and fluticasone propionate is in the range of 17-30 mm³, preferably in the range of 18-23 mm^{3,} most preferably in the range of 19-21 mm³ and each blister cavity having the volume described above is filled up to 25-100 %, preferably up to 70-100 %, most preferably up to 90-100 % of said volume in order to meet the specified needs for an effective inhalation. The lid and the base sheets of said blister pack are closed very tightly by any suitable method to provide impermeability.

According to the present invention, the lid and the base sheet constituting the blister package consist of several layers. Polymeric layers, aluminum foil and preferably Aclar® fluoropoylmer film are among the layers that form the lid and the base sheet.

Aclar® fluoropolymer film is a polymeric film which is used in blister packs and provides excellent moisture barrier. This chemically inert polymeric film does not cause any change in the taste of the formulation when it is in contact with the dry powder formulation. In addition, it easily constitutes a layered structure with the other polymeric layers which are composed of various polymers. It is appropriate to be transacted with heat.

In order to decrease the gas and moisture permeability of the layer, preferably desiccant agents are added to the polymeric layers to preserve the stability of the dry powder formulation stored in blisters that are arranged in an order on blister strips. Silica gel, zeolite, alumina, bauxite, anhydrous calcium sulfate, activated carbon and clay which have the property of water absorption can be given as examples to desiccant agents.

As it is common to use aluminum in lid and base sheets of high protection blister packs, aluminum is used both in the lid and the base sheets of the blister pack of the present invention in order to provide high moisture and gas protection. These aluminum foils must be thick enough to provide the desired protection for the stability of the moisture sensitive dry powder formulation stored in the blister cavity. Due to this reason, the thickness of the aluminum foil that is used in the lid and the base sheets of the blister pack is chosen to be in the range of 10 to 40 µm, preferably of 15 to 30 µm.

The polymeric layers in the lid and the base sheets of the blister pack mentioned in the present invention are made from the same or different polymers. The thickness of these polymeric layers varies according to the type of the polymeric substance used and its properties. Therefore, the thickness of the polymeric layer varies in the range of 15-60 µm, preferably of 20-35 µm depending on the type of the polymer used.

The inside layer of the blister cavity of the said blister pack which is in contact with the dry powder formulation is a polymeric layer because of the fact that some of the dry powder formulation sticks onto the inside layer of the blister cavity due to the porous structure of aluminum foil and electrostatic forces, and hence causes uncontrolled dosing.

According to the present invention, the polymers used to form the polymeric layers are preferably selected from a group comprising thermo-plastic polymers such as polyethylene, polypropylene, polystyrene, polyolefin, polyamide, polyvinyl chloride, polyurethane or other synthetic polymers.

In addition, the blisters which constitute the blister pack pertaining to the present invention can be in any shape as long as they have the properties described above.

According to the present invention, tiotropium bromide with water content less than or equal to 2.5% can be in crystal form and/or amorphous form or combination thereof.

According to the present invention, salmeterol xinafoate can be in the form of its solvates, hydrates enantiomers, diastereomers, racemates and/or in crystal form and/or amorphous form and/or a combination thereof.

According to the present invention, fluticasone propionate can be in the form of its solvates, hydrates, enantiomers or diastereoisomers, racemates and/or in crystal form and/or in amorphous forms and/or a combination thereof.

According to the present invention, the amount of tiotropium bromide water content less than or equal to 2.5% included in the drug formulation comprising tiotropium bromide with water content less than or equal to 2.5%, salmeterol xinafoate and fluticasone propionate is in the range of 1-40 µg, preferably 1-30 µg per dose.

According to the present invention, the amount of salmeterol xinafoate included in the drug formulation comprising tiotropium bromide with water content less than or equal to 2.5%, salmeterol xinafoate and fluticasone propionate is in the range of 1-600 µg, preferably 10-550 µg per dose.

According to the present invention, the amount of fluticasone propionate included in the drug formulation comprising tiotropium bromide with water content less than 2.5%, salmeterol xinafoate and fluticasone propionate is in the range of 1-600 µg, preferably 10-550 µg per dose.

The pharmaceutical composition mentioned in the present invention which comprises tiotropium bromide with water content less than 2.5%, salmeterol xinafoate and fluticasone propionate in dry powder form wherein;
- the particle size of tiotropium bromide with water content less than or equal to 2.5%, salmeterol xinafoate and fluticasone propionate is in the range of 1.5 to 4.5 µm and
- said active agents are present in the composition with the ratio 1 : 1 : 1 to 1 : 50 : 50 respectively and
- lactose which has a mean particle size less than 100 µm is used as carrier
can be used in the treatment of many respiratory diseases such as asthma, chronic obstructive pulmonary disorder (COPD) and allergic rhinitis. Accordingly, the respiratory diseases include, but not restricted to, allergic or non-allergic asthma at any phases, acute lung injury (ALI), acute respiratory distress syndrome (ARDS), exacerbation of airways hyperactivity, bronchiectasis, chronic obstructive pulmonary, airways or lung diseases (COPD, COAD or COLD) including emphysema and chronic bronchitis, pneumoconiosis, aluminosis, anthracosis, asbestosis, chalicosis, ptilosis, siderosis, silicosis, tabacosis and byssinosis. The treatment of said diseases may be prophylactic or symptomatic. In addition, the pharmaceutical composition pertaining to the present invention is used especially for the symptomatic treatment of asthma, allergic rhinitis and COPD.

A method for preparing the pharmaceutical composition according to the present invention comprises micronizing the tiotropium bromide with water content less than or equal to 2.5%, salmeterol xinafoate and fluticasone propionate, preferably by air jet mill, mixing micronized tiotropium bromide with water content less than or equal to 2.5%, salmeterol xinafoate, fluticasone propionate with lactose, and blending the composition to obtain a homogeneous dry powder mixture, and then filling the obtained dry powder mixture into capsules or blisters.

The combination of the pharmaceutical composition pertaining to the present invention can be explained with, but not restricted to, the examples given below.

### Example 1

So as to be used in a multiple dose inhaler, a dry powder drug formulation which is appropriate to be stored in blisters comprises 9 parts of tiotropium bromide with water content less than or equal to 2.5%, 50 parts of salmeterol xinafoate, 100 parts of fluticasone propionate having a mean particle size of 1.5-4.5 µm all of which are micronized in air jet mill and 10000 parts of lactose as carrier which has a particle diameter less than 100 µm.

The active substance tiotropium bromide with water content less than or equal to 2.5% given in this example includes its pharmaceutically acceptable amorphous and crystal forms thereof; salmeterol xinafoate includes its all pharmaceutically acceptable racemates, enantiomers or diastereomers, solvates, hydrates and/or amorphous and crystal forms and fluticasone propionate includes its all pharmaceutically acceptable solvates, hydrates and/or enantiomers and/or amorphous and crystal forms. Lactose in this example can optionally be added in a higher or a lower amount.

The amounts in Example 1 can be replaced by the amounts given in the table below and the example can be repeated.

| EXAMPLE | Amount of Tiotropum Bromide (Parts) | Amount of Salmeterol Xinafoate (Parts) | Amount of Fluticasone Propionate (Parts) | Amount of Lactose (Parts) |
|---|---|---|---|---|
| 2 | 11 | 12 | 200 | 16850 |
| 3 | 11 | 6 | 200 | 14960 |
| 4 | 21 | 6 | 400 | 15500 |
| 5 | 21 | 12 | 400 | 13500 |
| 6 | 11 | 12 | 200 | 15600 |
| 7 | 11 | 12 | 400 | 16120 |
| 8 | 11 | 6 | 400 | 15900 |
| 9 | 21 | 6 | 400 | 17000 |
| 10 | 21 | 12 | 200 | 13900 |
| 11 | 11 | 12 | 400 | 15600 |
| 12 | 11 | 12 | 400 | 14820 |
| 13 | 21 | 12 | 200 | 16250 |
| 14 | 11 | 6 | 400 | 16800 |
| 15 | 21 | 6 | 400 | 15900 |
| 16 | 21 | 6 | 400 | 13690 |
| 17 | 11 | 12 | 200 | 13780 |
| 18 | 11 | 12 | 200 | 14960 |
| 19 | 21 | 12 | 200 | 15360 |

### Example 20

A dry powder formulation which is appropriate for a gelatine capsule used in a capsule inhaler comprises 18 parts of tiotropium bromide with water content less than or equal to 2.5%, 50 parts of salmeterol xinafoate, 100 parts of fluticasone propionate having a mean particle size of 1,5 - 4,5 µm all of which are micronized in air jet mill and 5500 parts of lactose as carrier which has a particle diameter less than 100 µm.

The active agent tiotropium bromide with water content less than or equal to 2.5% given in this example includes its all pharmaceutically acceptable amorphous and/or crystal forms thereof; salmeterol xinafoate includes its all pharmaceutically acceptable racemates, enantiomers or diastereomers, solvates, hydrates and/or amorphous and/or crystal forms and fluticasone propionate includes its all pharmaceutically acceptable solvates, hydrates and/or enantiomers, and/or amorphous and/or crystal forms. Lactose given in this example can optionally be added in a higher or a lower amount. The capsule in this example is made of gelatin but it can optionally be made of chitosan, starch and/or starch derivatives, cellulose and/or cellulose derivatives or synthetic polymers.

The combination of the pharmaceutical composition pertaining to the present invention can be explained with, but not restricted to, the examples given below.

The amounts used in Example 20 can be replaced by the amounts given in the table below and the example can be repeated.

| EXAMPLE | Amount of Tiotropum Bromide (Parts) | Amount of Salmeterol Xinafoate (Parts) | Amount of Fluticasone Propionate (Parts) | Amount of Lactose (Parts) |
|---|---|---|---|---|
| 21 | 11 | 6 | 200 | 4500 |
| 22 | 11 | 12 | 200 | 4890 |
| 23 | 21 | 12 | 400 | 5020 |
| 24 | 11 | 12 | 400 | 4820 |
| 25 | 21 | 12 | 200 | 5260 |
| 26 | 21 | 12 | 200 | 5560 |
| 27 | 11 | 6 | 400 | 4960 |
| 28 | 11 | 6 | 400 | 6800 |
| 29 | 21 | 6 | 400 | 6050 |
| 30 | 11 | 12 | 400 | 6380 |
| 31 | 21 | 12 | 200 | 5930 |
| 32 | 21 | 12 | 300 | 3980 |
| 33 | 11 | 6 | 400 | 4330 |
| 34 | 21 | 6 | 400 | 4660 |
| 35 | 21 | 12 | 200 | 4600 |
| 36 | 11 | 12 | 200 | 4900 |
| 37 | 11 | 6 | 400 | 5230 |
| 38 | 21 | 6 | 400 | 5360 |

## Claims

1. A pharmaceutical composition for use in the symptomatic and/or prophylactic treatment of respiratory diseases comprising the simultaneous or sequential administration of tiotropium bromide with water content less than or equal to 2.5%, salmeterol xinafoate and fluticasone propionate in dry powder form wherein
o the mean particle size of tiotropium bromide with water content less than or equal to 2.5%, salmeterol xinafoate and fluticasone propionate is in the range of 1.5 to 4.5 µm and
o said active agents are present in the composition with the ratio of 1 : 1 : 1 to 1 : 50 : 50 respectively and
o lactose which is used as carrier has a mean particle size less than 100 µm.

2. The pharmaceutical composition for use according to claim 1, wherein said composition comprises tiotropium bromide with water content less than or equal to 2.5%, salmeterol xinafoate, fluticasone propionate and lactose.

3. The pharmaceutical composition for use according to claim 1, wherein tiotropium bromide with water content less than or equal to 2.5%, salmeterol xinafoate, fluticasone propionate are combined in a single dosage form.

4. The pharmaceutical composition for use according to claim 1, wherein the amount of salmeterol xinafoate and/or its pharmaceutically acceptable solvates, hydrates enantiomers, diastereomers, racemates is in the range of 1-600 µg per dose.

5. The pharmaceutical composition for use according to claim 1, wherein the amount of fluticasone propionate and/or its pharmaceutically acceptable solvates, hydrates, enantiomers or diastereoisomers, racemates is in the range of 1-600 µg per dose.

6. The pharmaceutical composition for use according to claim 1, wherein the amount of tiotropium bromide with water content less than or equal to 2.5% is in the range of 1-40 µg per dose.

7. The pharmaceutical composition for use according to claim 1, wherein lactose which has a mean particle size less than 100 µm is used as a mixture of particles having two different mean particle sizes.

8. The pharmaceutical composition for use according to claim 7, wherein lactose which has a mean particle size less than 100 µm is present as a mixture of particles having a mean particle size less than 10 µm and particles having a mean particle size in the range of 10 µm to 100 µm.

9. The pharmaceutical composition for use according to claim 8, wherein the ratio of lactose which has a mean particle size less than 10 µm (fine) to lactose which has a mean particle size in the range of 10 µm to 100 µm (coarse) is in the range of 1:1 to 1:25.

10. The pharmaceutical composition for use according to claim 8, wherein the ratio of lactose which has a mean particle size less than 10 µm (fine) to lactose which has a mean particle size in the range of 10 µm to 100 µm (coarse) is in the range of 1:1 to 1:10.

11. The pharmaceutical composition for use according to claim 8, wherein the ratio of lactose which has a mean particle size less than 10 µm (fine) to lactose which has a mean particle size in the range of 10 µm to 100 µm (coarse) is in the range of 1:1,5 to 1:5.

12. The pharmaceutical composition for use according to claim 1, wherein said pharmaceutical composition in dry powder form can be stored in capsules, reservoirs or blister packs.

13. The pharmaceutical composition comprising tiotropium bromide with water content less than or equal to 2.5%, salmeterol xinafoate and fluticasone propionate in dry powder form wherein
o the mean particle size of tiotropium bromide with water content less than or equal to 2.5%, salmeterol xinafoate and fluticasone propionate is in the range of 1.5 to 4.5 µm and
o said active agents are present in the composition with the ratio of 1 : 1 : 1 to 1 : 50 : 50 respectively and
o lactose which is used as carrier has a mean particle size less than 100 µm is administered simultaneously or sequentially for use in the treatment of respiratory diseases especially asthma, allergic rhinitis, chronic obstructive pulmonary disease (COPD).

14. The pharmaceutical composition for use according to claim 1, wherein said composition is used for simultaneous administration in the symptomatic and/or prophylactic treatment of respiratory diseases.

## Patentansprüche

1. Pharmazeutische Zusammensetzung zur Verwendung bei der symptomatischen und / oder prophylaktischen Behandlung von Erkrankungen der Atemwege, enthaltend die gleichzeitige oder sequentielle Anwendung von Tiotropiumbromid mit einem Wassergehalt weniger als oder gleich zu 2.5%, Salmeterolxinafoat und Fluticasonpropionat in trockener Pulverform, wobei
die mittlere Teilchengröße von Tiotropiumbromid mit einem Wassergehalt weniger als oder gleich zu 2.5%, Salmeterolxinafoat und Fluticasonpropionat im Bereich von 1.5 bis 4.5 µm ist und
die genannten Wirkstoffe in der Zusammensetzung mit dem Verhältnis von 1: 1: 1 bis 1: 50: 50 jeweils vorhanden sind und
Laktose, die als Träger verwendet wird, eine mittlere Teilchengröße weniger als 100 µm hat.

2. Pharmazeutische Zusammensetzung zur Verwendung nach Anspruch 1, wobei die erwähnte Zusammensetzung Tiotropiumbromid mit einem Wassergehalt weniger als oder gleich zu 2.5%, Salmeterolxinafoat, Fluticasonpropionat und Laktose enthält.

3. Pharmazeutische Zusammensetzung zur Verwendung nach Anspruch 1, wobei Tiotropiumbromid mit einem Wassergehalt weniger als oder gleich zu 2.5%, Salmeterolxinafoat und Fluticasonpropionat in einer einzelnen Dosierungsform kombiniert sind.

4. Pharmazeutische Zusammensetzung zur Verwendung nach Anspruch 1, wobei die Menge von Salmeterolxinafoat und / oder dessen pharmazeutisch verträglichen Solvate, Hydrate, Enantiomere, Diastereomere und Racemate im Bereich von 1-600 µg pro Dosis liegt.

5. Pharmazeutische Zusammensetzung zur Verwendung nach Anspruch 1, wobei die Menge von Fluticasonpropionat und / oder dessen pharmazeutisch verträglichen Solvate, Hydrate, Enantiomere, Diastereomere und Racemate im Bereich von 1-600 µg pro Dosis liegt.

6. Pharmazeutische Zusammensetzung zur Verwendung nach Anspruch 1, wobei die Menge von Tiotropiumbromid mit einem Wassergehalt weniger als oder gleich zu 2.5% im Bereich von 1-40 µg pro Dosis liegt.

7. Pharmazeutische Zusammensetzung zur Verwendung nach Anspruch 1, wobei Laktose, die eine mittlere Teilchengröße weniger als 100 µm hat, als eine Mischung von Partikeln verwendet wird, die zwei unterschiedlichen mittleren Teilchengröße aufweist.

8. Pharmazeutische Zusammensetzung zur Verwendung nach Anspruch 7, wobei Laktose, die eine mittlere Teilchengröße weniger als 100 µm hat, als eine Mischung von Partikeln mit einer mittleren Teilchengröße weniger als 10 µm und mit einer mittleren Teilchengröße im Bereich von 10 µm bis 100 µm vorhanden ist.

9. Pharmazeutische Zusammensetzung zur Verwendung nach Anspruch 8, wobei das Verhältnis von Lactose, die eine mittlere Teilchengröße weniger als 10 µm (fein) hat, zu der Laktose, die eine mittlere Teilchengröße in dem Bereich von 10 µm bis 100 µm (grob) hat, im Bereich von 1:1 bis 1:25 liegt.

10. Pharmazeutische Zusammensetzung zur Verwendung nach Anspruch 8, wobei das Verhältnis von Lactose, die eine mittlere Teilchengröße weniger als 10 µm (fein) hat, zu der Laktose, die eine mittlere Teilchengröße in dem Bereich von 10 µm bis 100 µm (grob) hat, im Bereich von 1:1 bis 1:10 liegt.

11. Pharmazeutische Zusammensetzung zur Verwendung nach Anspruch 8, wobei das Verhältnis von Lactose, die eine mittlere Teilchengröße weniger als 10 µm (fein) hat, zu der Laktose, die eine mittlere Teilchengröße in dem Bereich von 10 µm bis 100 µm (grob) hat, im Bereich von 1:1,5 bis 1:5 liegt.

12. Pharmazeutische Zusammensetzung zur Verwendung nach Anspruch 1, wobei die genannte Zusammensetzung in trockener Pulverform in Kapseln, in den Stauseen oder Blistern aufbewahrt werden kann.

13. Pharmazeutische Zusammensetzung enthaltend Tiotropiumbromid mit einem Wassergehalt weniger als oder gleich zu 2.5%, Salmeterolxinafoat und Fluticasonpropionat in trockener Pulverform, wobei
die mittlere Teilchengröße von Tiotropiumbromid mit einem Wassergehalt weniger als oder gleich zu 2.5%, Salmeterolxinafoat und Fluticasonpropionat im Bereich von 1.5 bis 4.5 µm ist und
die genannten Wirkstoffe in der Zusammensetzung mit dem Verhältnis von 1: 1: 1 bis 1: 50: 50 jeweils vorhanden sind und
Laktose, die als Träger verwendet wird, eine mittlere Teilchengröße weniger als 100 µm hat und gleichzeitig oder nacheinander für die Verwendung bei der Behandlung von Atemwegserkrankungen insbesondere wie Asthma, allergische Rhinitis, chronischobstruktive Lungenerkrankung (COPD) eingesetzt wird.

14. Pharmazeutische Zusammensetzung zur Verwendung nach Anspruch 1, wobei die erwähnte Zusammensetzung für die gleichzeitige Anwendung in der symptomatischen und/oder prophylaktischen Behandlung von Atemwegserkrankungen eingesetzt wird.

## Revendications

1. La composition pharmaceutique utilisée pour le traitement symptomatique et/ou prophylectique de maladies respiratoires comprenant l'administration simultanée ou séquentielle de bromure de tiotropium avec une teneur en eau inférieur ou égale a 2.5%, xinafoade de salmeterol et propionate de fluticasone sous forme de poudre sèche dans laquelle
• La dimension moyenne de particule de bromure de tiotropium avec une teneur en eau inferieur ou égale a 2.5 %, xinafoate de sarneterol et propionate de fluticasone dans l'intervalle de 1.5 a 4.5 µm et,
• Lesdits agents actifs sont présents dans la composition avec une ratio de 1: 1: 1 à 1 : 50 : 50 recpectivement.
• Lactose qui est utilisé oomme un support a une dimension moyenne de particule inferieure à 100 µm.

2. La composition pharmaceutique utilisée selon la revendication 1 dans laquelle ladite composition comprend du bromure de tiotropium avec une teneur en eau inferieure ou égale à 2.5%, xinafoate de salmeterol, propionate de fluticasone et lactose.

3. La composition pharmaceutique utilisée selon la revendication 1 dans laquelle la bromure de tiotropium avec une teneur en eau infériuere ou égale à 2.5%, xinafoate de salmeterol et propionate de fluticasone sont combinés sous une forme de dosage unique.

4. La composition pharmaceutique utilisée selon la revendication 1 dans laquelle la quantité de xinafoate de salmeterol et/ou ses solvantes pharmaceyutiquement acceptables, les hydrates, enantiomères, diastéréomères, racemates est dans la gamme de 1-600 µg par dosage.

5. La composition pharmaceutique utilisée selon la revendication 1 dans laquelle la quantité de propionate de fluticasone et/ou ses solvantes pharmaceyutiquement acceptables, les hydrates, enantiomères, diastéréomères, racemates est dans l'intervalle de 1-600 µg par dosage.

6. La composition pharmaceutique utilisée selon la revendication 1 dans laquelle la quantité de bromure de tiotropium avec une teneur en eau inférieur ou égale à 2.5%, est dans l' intervalle de 1-40 µg par dosage.

7. La composition pharmaceutique utilisée selon la revendication 1 dans laquelle le lactose qui a une moyenne dimension de particule 100 µm, est utilisé comme un mélange de particules ayant deux différentes dimensions moyens de particules.

8. La composition pharmaceutique utilisée selon la revendication 7 dans laquelle le lactose qui a une moyenne dimension de particule 100 µm, est présent sous forme d'un mélange de particules ayant une dimension moyenne inféerieure à 10 µm et des particules ayant une dimension moyenne de particule dans l'intervalle de 10 µm à 100 µm.

9. La composition pharmaceutique utilisée selon la revendication 8, dans laquelle la ratio du lactose a une dimension moyenne de particule inferieur à 10 µm (fine) au lactose qui a une dimension moyenne de particules dans l'intervalle de 10 µm à 100 µm (coarse) est dans l'intervalle de 1:1 à 1:25.

10. La composition pharmaceutique utilisée selon la revendication 8, dans laquelle la ratio du lactose a une dimension moyenne de particule inferieur à 10 µm (fine) au lactose qui a une dimension moyenne de particules dans l'intervalle de 10 µm à 100 µm (coarse) est dans l'intervalle de 1:1 à 1:10.

11. La composition pharmaceutique utilisée selon la revendication 8, dans laquelle la ratio du lactose a une dimension moyenne de particule inferieur à 10 µm (fine) au lactose qui a une dimension moyenne de particules dans l'intervalle de 10 µm à 100 µm (coarse) est dans l'intervalle de 1:1,5 à 1:5.

12. La composition pharmaceutique utilisée selon la revendication 1, dans laquelle ladite composition parmaceutique sous forme de poudre sèche peut etre entrposé dans des capsules, des réservoirs ou des blisters.

13. La composition pharmaceutique comprenant le bromure de tiotropium avec la teneur en eau inferieur ou égale à 2.5%, le xinafoate de salmétérol et de propionate de fluticasone en forme poudre sèche dans laquelle;
• La dimension moyenne des particules de bromure de tiotropium avec une teneur en eau inferieur ou égale à 2.5%, le xinafoate de salmétérol et propionate de fluticasone sont dans l'intervalle de 1.5 à 4.5 µm et
• Lesdits agents actifs sont présents dans la composition avec la ratio de 1:1:1 à 1:50:50 respectivement et
• Le lactose qui est utilisé comme porteur a une dimension moyenne de particules inferieur à 100 µm est administré simultanément ou séquentiellement pour utilisation dans le traitement des maladies respiratoires, en particulier de l'asthme, la rhinite allergique, bronchopneumopathie chronique obstructive (BPCO).

14. La composition pharmaceutique pour utilisation selon la revendication 1, dans laquelle ladite composition est utilisée pour une administration simultanée dans le traitement symptomatique et / ou prophylactique de maladies respiratoires.
